(19) **Europäisches Patentamt / European Patent Office / Office européen des brevets**

(11) **EP 1 535 584 B1**

(12) **EUROPEAN PATENT SPECIFICATION**

(45) Date of publication and mention
of the grant of the patent:
**23.01.2008 Bulletin 2008/04**

(51) Int Cl.:
***A61B 18/22*** *(2006.01)*

(21) Application number: **04257302.2**

(22) Date of filing: **24.11.2004**

(54) **Energy delivery device with self-heat calibration**

Energieabgabegerät mit Selbstaufheizungskalibration

Dispositif de distribution d'énergie avec calibration d'auto-chauffage

(84) Designated Contracting States:
**DE FR GB IT**

(30) Priority: **25.11.2003 US 721111**

(43) Date of publication of application:
**01.06.2005 Bulletin 2005/22**

(73) Proprietor: **ETHICON ENDO-SURGERY**
**Cincinnati,**
**Ohio 45242 (US)**

(72) Inventors:
 • **Ritchie, Paul G.**
 **Loveland, Ohio 45140 (US)**

 • **Speeg, Trevor**
 **Williamsburg, Ohio 45176 (US)**

(74) Representative: **Tunstall, Christopher Stephen et al**
 **Carpmaels & Ransford**
 **43-45 Bloomsbury Square**
 **London WC1A 2RA (GB)**

(56) References cited:
 **US-A- 4 564 012**         **US-A1- 2002 081 871**
 **US-B1- 6 522 806**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

<u>Field of the Invention</u>

The present invention relates generally to a system for applying energy to human tissue, and more particularly, to such a system having information storage capability. The present invention also relates to an energy delivery device, and methods for use thereof, having capabilities to correct for inherent energy self-absorption properties thereby properly calibrating the energy delivery device for optimal clinical effect.

<u>Background of the Invention</u>

Currently surgeons frequently employ medical instruments which incorporate energy technology in the treatment of benign prostatic hyperplasia, which is commonly referred to as BPH. BPH is a condition of an enlarged prostate gland, in which the gland having BPH typically increases multiple times beyond its normal size. Methods generally known as Interstitial Thermotherapy (ITT), and specifically Laser Interstitial Thermotherapy, sometimes referred to as LITT, have been widely used in the treatment of such human tissue. ITT utilizes energy delivery devices, often in the form of LITT using laser light sources, are employed by surgeons to treat this condition using optical fibers that emit light radially in a predictable and controlled manner. The goal of LITT is to diffuse light into the human tissue in a controlled manner causing the intended portions of such human tissue to die. Similar devices are also used for Photo-Dynamic Therapy (PDT), wherein a light-activated pharmaceutical agent is used in combination with diffusing fibers to treat human diseases.

A common problem of such diffusing fiberoptic technology is that the diffusing section of the energy delivery device tends to absorb some of the emitted light. This absorption of light is manifested by a phenomena known as self-heating. This phenomena causes the diffusing section to slightly increase in temperature or to heat itself up. Self-heating is believed to be due to the energy self-absorption properties of the energy delivery device and occurs when a portion of the light being diffused through the diffusing section is absorbed by the energy delivery device itself causing the temperature of the diffusing section of the energy delivery device to increase. These energy self-absorption properties can be influenced by the design, composition, or construction of the various layers and constituents in the energy delivery device.

Some energy delivery devices are made to absorb energy so that they do heat up. Such devices are often used as cutting or ablating implements. Ablating devices are similar to those that contain sapphire tipped optical fibers and which are intended to absorb energy in order to heat up wherein the heated optical fiber allows the device to cut or burn through human tissue. These ablating devices are typically used just like a fiberoptic scalpel. Ablating devices are distinctly different from the diffusing energy delivery devices of the present invention and are used for wholly different purposes than the diffusing energy delivery device described herein. Diffusing type energy delivery devices are used to diffuse energy by allowing the energy to pass into the human tissue itself and are not intended to actually cut through the human tissue or to absorb any of the available energy.

During the treatment of human tissue by LITT, accurately controlling the amount of energy diffused through the optical fiber and absorbed by the human tissue is critical. Even minor variations in the temperature at the location being treated can change the therapeutic effects of treatment. The self-heating phenomena can lead to the under-treatment of the human tissue in the area being treated. This occurs because the system indicates the temperature is higher than it really is, so it reduces power level sooner and under treats the human tissues. Such uneven or ineffective treatment can result in detrimental clinical outcomes. Self-heating can also have adverse effects on the energy delivery device itself including failure, melting, or separation of the optical fiber.

Some devices attempt to address the issue of self-heating by teaching the use of a particularly configured energy delivery device that helps to minimize the amount of self-heating. U.S. Patent No. 5,074,632 issued to Potter discloses a fiber optic diffuser design intended to produce low loss diffusers with material having minimal absorbance of the light in the wavelength range of the light source.

The art also discloses numerous mechanisms for measuring temperature levels during use that include feedback loops in an attempt to control the temperature of the fiberoptic device. U.S. Patent No. 4,695,697 issued to Kosa discloses a control system for an optical fiber laser power delivery system utilizing a synthetic sapphire lens having temperature dependent fluorescing properties useful in generating a signal in a feedback control system. U.S. Patent No. 4,476,512 issued to Sunago et al. discloses a monitor device for use in a laser system transmitting laser light through an optical fiber including a heat-sensitive element which produces an output to monitor the laser system. Another method for laser surgery, U.S. Patent No. 5,057,099 issued to Rink, discloses a temperature control device having a light sensitive diode or some other photosensitive detector designed to respond to infrared radiation wherein a temperature control device is associated with a laser which monitors the temperature of the surgical tool and governs the laser power output to achieve a desired temperature level.

US 4,564,012 discloses a laser surgical equipment consisting of a laser oscillator oscillating a laser output, an optical waveguide connected to the laser oscillator and transmitting the laser output, a hand piece connected to the optical waveguide and radiating the laser output to a diseased part to be radiated, a detector for detecting the laser output from the hand piece, a comparator for comparing the output of the detector with a predetermined value and a controller for controlling the laser oscillator by the output of the comparator and for setting the laser output of the hand piece to the predetermined value.

While some of these designs attempt to minimize the amount of self-heating, other designs simply measure a temperature at the point of contact and attempt to maintain a desired energy setting associated therewith. These attempts fail to take into consideration that all diffuser type energy delivery devices have some inherent amount of self-heating due to the intrinsic properties of the materials and components from which the energy delivery devices are constructed. None of the prior art devices attempt to determine the magnitude of this self-heating characteristic nor do any attempt to make a correction in their temperature measurements for this inherent phenomena. Temperature control systems that do not take into consideration this self-heating phenomena can lead to inaccurate temperature readings even when temperature sensors are utilized. Such inaccuracies in temperature can lead to mistreatment of the human tissue.

Consequently, there is a need for medical treatment systems and devices that provide for the measurement and adjustment or correction for the energy self-absorption properties that result in self-heating characteristics in order to assure accurate, reliable and repeatable human tissue temperature control for more efficacious treatment results during such surgical procedures. There is also a need for methods and processes that accurately, reliably and repeatably account for these energy self-absorption properties that result in these self-heating characteristics arising during surgical treatments.

Summary of the Invention

The present invention provides an energy delivery device according to claims 1 to 10.

The self-heating characteristic can be associated with a particular power level setting of the medical treatment system. Typically, the self-heating characteristic is a function of a power level and the function can be modeled by an equation. In one embodiment, the equation is a linear equation. However, the equation could be a non-linear equation, quadratic equation, or the like.

A connector can be utilized to connect the energy delivery device to an energy generator. Both the optical fiber and the memory device can be attached to the connector. The optical fiber has a proximal end and a distal end. The proximal end can be attached to the connector and the distal end can be in the form of a penetrating tip. The optical fiber further includes a diffusing section located adjacent to the distal end. The energy delivery device according to the present invention has a temperature sensor adjacent to the diffusing section wherein the temperature sensor includes alexandrite particles or some other temperature sensing mechanism.

The invention also provides a memory device for use with an energy delivery device in combination with an optical fiber as recited in claim 11.

There is also disclosed a method for producing a medical treatment system for the treatment of human tissue wherein the energy delivery device includes a memory device. This method comprises the steps of: measuring at least one self-heating characteristic; determining one or more calibration parameters, indicative of the self-heating characteristic; and storing the calibration parameters in the memory device. Additional steps can be provided such as: reading the calibration parameter from the memory device; setting a power level for the medical treatment system; reading a measured temperature; calculating a corrected temperature value using the calibration parameter and the measured temperature; and adjusting the power level in response to the corrected temperature value.

In such a method, the optical fiber can be included wherein the self-heating characteristic is specifically associated with the optical fiber. The optical fiber can also include a distal end and a proximal end. The method of producing a medical treatment system can further include the step of reading a measured temperature taken at the distal end of the optical fiber. The calibration parameter is derived from the self-heating characteristic of the optical fiber and the self-heating characteristic results from the energy self-absorption properties of the optical fiber.

A particularly preferred method of producing a medical treatment system for the treatment of human tissue wherein the medical treatment system includes a memory device includes the steps of: measuring a self-heating characteristic; determining a calibration parameter indicative of the self-heating characteristic; storing the calibration parameter in the memory device; reading the calibration parameter from the memory device; setting a power level for the medical treatment system; reading a measured temperature; calculating a corrected temperature value using the calibration parameter and the measured temperature; and adjusting the power level in response to the corrected temperature value.

The present invention thus provides an energy delivery device for applying energy to human tissue, which includes an optical fiber and a memory device wherein the memory device has data programmed therein that is specifically associated with the energy self-absorption properties of the optical fiber.

Additional advantages and features of the present invention will become more apparent from the following detailed description which may be best understood with reference to and in conjunction with the accompanying drawings.

Brief Description of the Drawings

FIG. 1 is an isometric view of a medical treatment system, including an energy generator and an energy delivery device according to an embodiment of the present invention;
FIG. 2 is an isometric view of the energy generator of FIG. 1 with the cover removed for clarity;
FIG. 3 is an isometric view of the connector of FIG. 1;
FIG. 4 is a sectional view taken in side elevation along the centerline of the connector shown in FIG. 3;
FIG. 5 is a plan view showing an opposite side of the printed circuit board of FIG. 4;
FIG. 6 is a sectional view taken in side elevation of an optical fiber of FIG. 1;
FIG. 7 is a graphical plot of self-heating characteristic versus power level for optical fibers according to the present invention; and
FIG. 8 is a flow chart illustrating a method for use of an energy delivery device in accordance with the present invention.

Detailed Description of the Invention

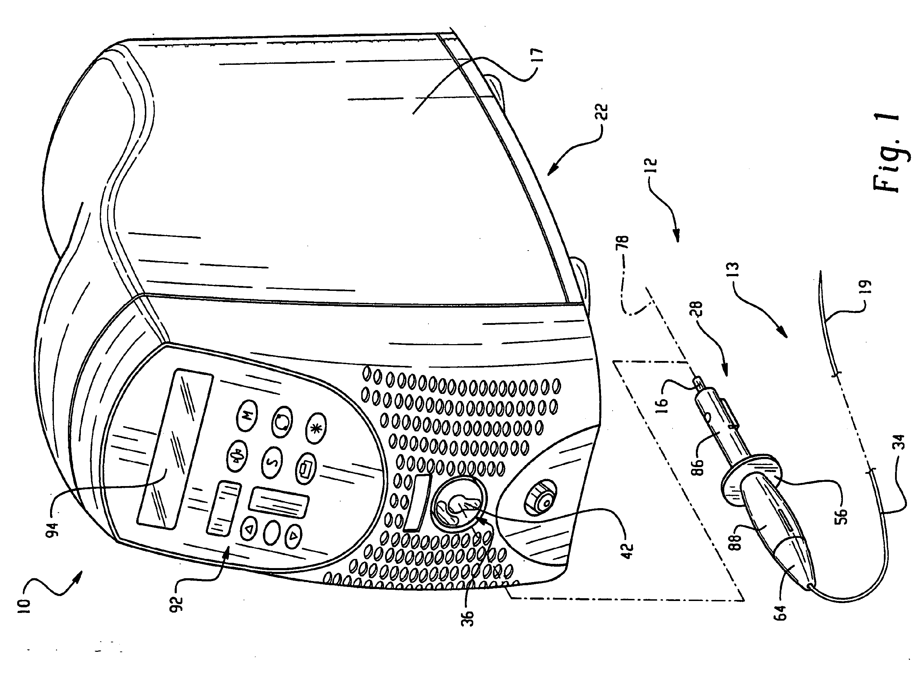

In this description of preferred embodiments, "means for generating energy" and "energy generator", "generator" or the like, can be used interchangeably and, similarly, "energy delivering means" and "energy delivery device", "delivery device" or the like, can be used interchangeably unless otherwise specified. Additional terms will be used in the same manner, as will be clear to the reader. Further, the terms "proximal" and "distal" are used to refer to relative locations nearest to and farthest from, respectively, the connector 28 of the energy delivery device 12 of the medical treatment system 10, as shown in FIG. 1. These conventions are adopted merely by way of convenience, not by way of limitation.

According to an embodiment of the present invention shown in FIG. 1, medical treatment system 10 for transferring diffused light energy to human tissue which includes energy generator 22 and energy delivery device 12, is illustrated in a disconnected configuration in FIG. 1. An energy generator 22 is provided with medical treatment system 10 to generate energy in the form of laser light. Energy generator 22 could be any means for generating energy or a generator for many different types of energy such as, for example, laser light energy, infrared energy, radio frequency energy, microwave energy, ultrasound energy or any other energy suitable for the treatment of human tissue. By way of example, a means for generating ultrasonic energy may be the Ultracision Harmonic Scalpel commercially available from Ethicon Endo-Surgery Inc., of Cincinnati, Ohio, and a means for generating radio-frequency energy may be any of a variety of surgical generators, such as the ICC 350 Electrosurgical Generator commercially available from Erbe USA, Inc., of Marietta, Ga. Preferably, energy generator 22 is a portable diode based laser, and most preferably, the Indigo® Optima laser system commercially available from Ethicon Endo-Surgery, Inc.

A cover 17 shields interior components of energy generator 22, and a connector housing 36 resides within a front portion of cover 17. The front of connector housing 36 is exposed to the exterior. Medical treatment system 10 further includes an energy delivery device 12 having connector 28 at its proximal end and optical fiber 13 at its distal end. The optical fiber 13 of energy delivery device 12 extends from connector 28 to light-diffusing section 19. Optical fiber 13 could be associated with any energy delivery device 12 capable of delivering useful energy such as, for example, laser light energy, infrared energy, radio frequency energy, microwave energy, ultrasound energy or any other energy suitable for the treatment of human tissue. Energy delivery device 12 could be any means for delivering energy or any device capable of delivering many types of useful energy from the energy generator 22.

Energy delivery device 12 is attachable to connector housing 36 by inserting connector 28 through an opening 42 in connector housing 36 to lock the connector 28 in position. Connector 28 inserts into connector housing 36 and locks into connector housing 36 by rotation about a longitudinal axis 78. In one embodiment, energy delivery device 12 may be usage-limited, such as a disposable delivery device, for delivering energy from an energy generator 22 to human tissue one time only or for a set number of times. In this embodiment, energy delivery device 12 can be removed from energy generator 22 by unlocking connector 28 from connector housing 36 by rotation about a longitudinal axis 78 in a direction opposite the locking rotation.

As shown in FIG. 1, the energy generator 22 may include a keypad 92 on cover 17 for user interface and input of data. The energy generator 22 may also include a display screen 94 on cover 17 for the display of data, warnings, or other information.

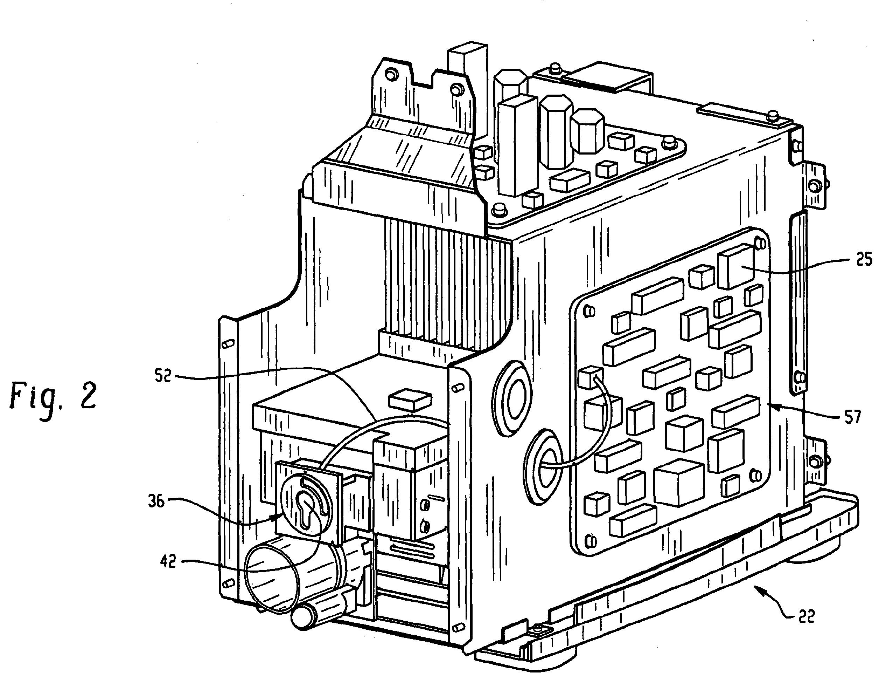

FIG. 2 depicts energy generator 22 with cover 17 removed to expose interior portions of energy generator 22. Conductor cable 52 electrically joins connector housing 36 to controller board 57 on energy generator 22. Located on controller board 57 is a computer in the form of main processor 25, which receives and processes electronic signals to control the operation of medical treatment system 10. Main processor 25 can be, for example, a microprocessor. Signals from electronic components within energy delivery device 12 communicate via conductor cable 52 with controller board 57 and main processor 25. Additionally, the main processor 25 can be operatively connected to the keypad 92 and the display screen 94.

In operation, the main processor 25 directs the energy application process according to instructions from the user via the keypad 92 or programmed instructions from the energy delivery device 12, as further described herein. The main processor 25 communicates information concerning the process to the display screen 94 for observation by the user. Should the user find the information concerning the process undesirable, for example, unsafe to the patient undergoing treatment, he or she may override the operating instructions via the keypad 92.

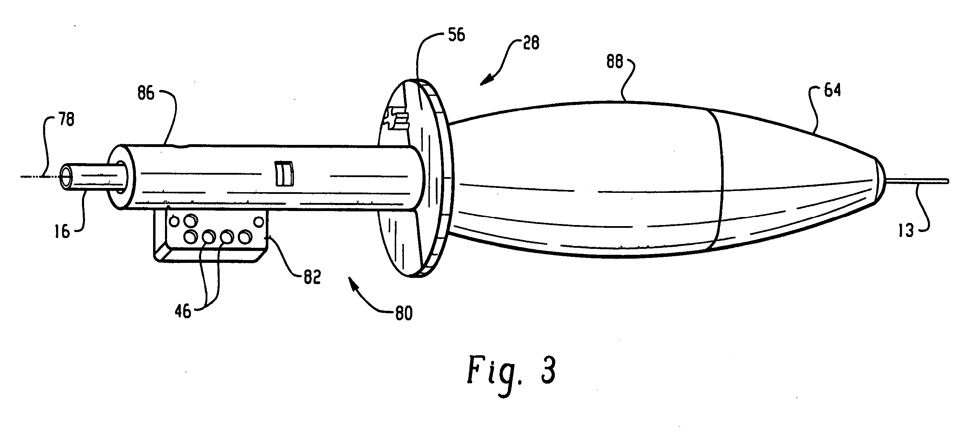

As shown in FIG. 3, connector 28 possesses a handle portion 88, shaped for easy grasping by the user, and capped on the distal end with a boot 64. Optical fiber 13 extends distally from the boot 64. A barrel 86 continues proximately from handle portion 88. A connector face 56 separates barrel 86 from handle portion 88. Attached to barrel 86 is a flange 82 radially extending from longitudinal axis 78. Flange 82 includes contact pad access openings 46 placed on a large side of flange 82. An axial gap 80 separates the distal end of flange 82 from connector face 56. Ferrule 16 is located within connector 28 and a portion of ferrule 16 protrudes from the proximate end of barrel 86. Ferrule 16 is one form of an energy transfer attachment for transferring energy from energy generator 22 to energy delivery device 12 for medical treatment. Opening 42 on connector housing 36 allows entrance of barrel 86 of connector 28 to operatively connect the energy delivery device 12 to the energy generator 22.

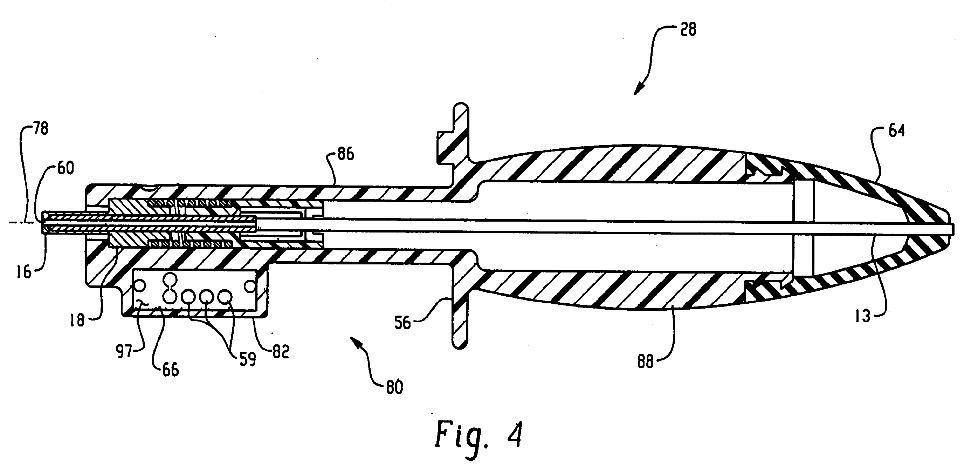

A cross sectional view of connector 28 is shown in FIG. 4 depicting the interior portions of connector 28. Ferrule 16 has a passageway 60 through the center thereof to admit light energy generated by energy generator 22 into optical fiber 13. The passageway 60 in ferrule 16 is coaxial with longitudinal axis 78. The interior of handle portion 88 engages enlarged portion 18 of ferrule 16 and boot 64 surrounds and retains optical fiber 13 as it emerges from handle portion 88 of connector 28. Printed circuit board 66 within flange 82 is also illustrated with mating surface 97. Printed circuit board 66 can be insert-molded into flange 82 leaving only contact pads 59 open to the exterior through access openings 46. Connector 28 is preferably molded of non-conductive material such as plastic.

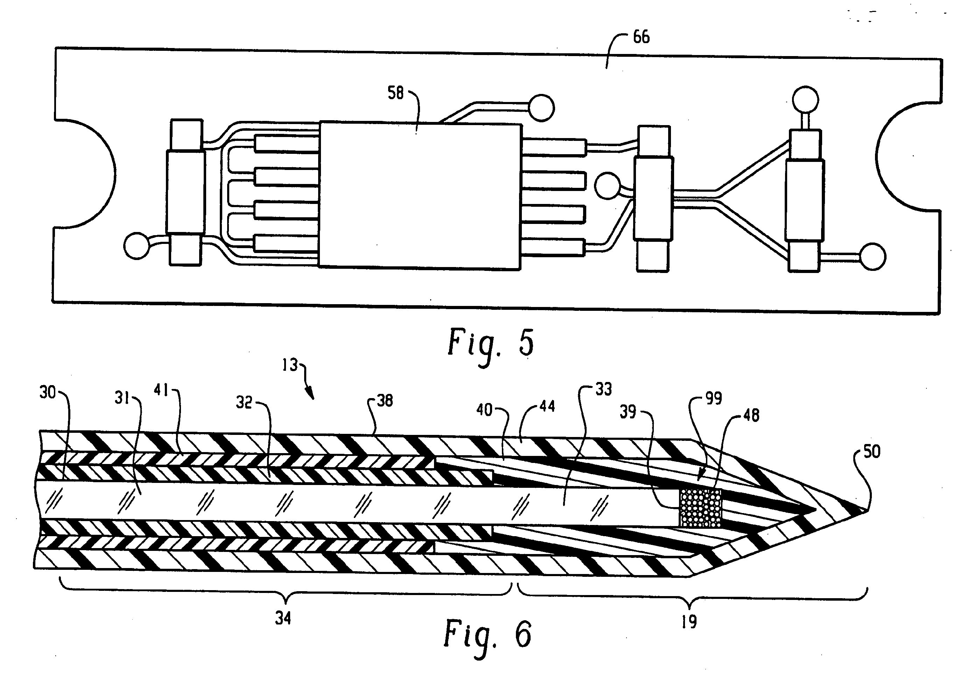

FIG. 5 depicts the side of printed circuit board 66 opposite that shown in FIG. 4. A memory device 58 resides on the side of printed circuit board 66 opposite mating surface 97 and is in electrical communication with contact pads 59. Memory device 58 can be, for example, an electronic erasable programmable read-only memory device (EEPROM) and can store information useful to the operation of energy delivery device 12 and medical treatment system 10.

With connector 28 in the locked position, memory device 58 can communicate electrically with main processor 25 on controller board 57 through contact pads 59 and conductor cable 52. Information within memory device 58 may now be accessed by main processor 25 and vice versa.

While the memory device 58 has been described as an EEPROM, which may store a significant amount of data, it may alternatively be any of a variety of digital, optical, or magnetic memory storage devices or integrated circuits providing memory capability. Of course, the entire set of data or information need not be stored in a single memory device 58, multiple memory devices 58 can be used in accordance with the present invention. Further, while the memory device 58 has been described as being mounted on printed circuit board 66 which is inset molded on flange 82, it is understood that printed circuit board 66 or memory device 58 can alternatively be externally mounted or even a wholly separate assembly that engages energy generator 22 or energy delivery device 12 via a separate electrical connection or some other method. Additionally, while the data exchange between the memory device 58 and the energy generator 22 has been described as possibly being accomplished via electrical means, it may alternatively be accomplished via magnetic, infrared, radio frequency or even optical means. These alternatives and others which may be arrived at by one of ordinary skill in the art without undue experimentation are contemplated as being within the scope of the present invention.

The information stored or programmed into memory device 58 may include calibration parameters, identification numbers, expiration date, and prior usage history of energy delivery device 12 along with various other data relating to optical fiber 13. This will be described in more detail below.

Energy delivery device 12 adapted to be employed for these purposes typically extends from a connector 28 to at least the distal end of the optical fiber 13. Preferably, the energy delivery device 12 includes a means for diffusing energy from the energy delivery device 12 to the human tissue at or near its distal end. In particular, medical treatment system 10, with energy delivery device 12, can be used to apply laser light energy to human tissue for therapeutic treatment of the human tissue, for example, for treatment of diseases such as BPH using LITT.

Now referring to FIG. 6, an energy delivery device 12 according to one embodiment of the present invention, includes an optical fiber 13 comprising a diffuser or light-diffusing section 19 at its distal end and a non-diffusing or light-transmitting portion 34 extending toward its proximal end. In light-transmitting portion 34 of optical fiber 13, a cladding 32 and the proximal portion of a sheath or sleeve 38 radially surround the proximal portion 30 of core 31. Optical fiber 13 may have a jacket or buffer layer 41 arranged to extend circumferentially between the cladding 32 and the sleeve 38. The material used to form the cladding 32 has an index of refraction lower than the index of refraction of the material used to create the glass or core 31 so as to contain the light within the core 31 throughout the length of the light-transmitting portion 34. In light-diffusing section 19 of optical fiber 13, the core 31 extends beyond its proximal portion 30 through a distal portion 33 to the distal end 39 thereof. The distal portion 33 of the core 31, which is employed to diffuse light, is surrounded by an optical coupling layer 40 and the distal portion 44 of the sleeve 38 thereby forming the light-diffusing section 19 without the cladding 32 of the light-transmitting portion 34.

A material having an index of refraction higher than the index of refraction of the core 31 forms the optical coupling layer 40. Preferably, UV50 Adhesive, commercially available from Chemence, Incorporated, in Alpharetta, Ga., is the adhesive used to produce the optical coupling layer 40.

The sleeve 38 can extend distally past the distal end 39 of the core 31 and may be configured to form a sharp or pointed penetrating tip 50. Penetrating tip 50 is capable of piercing through human tissue in order to assist medical procedures. In a preferred embodiment, sleeve 38 constitutes one continuous piece, more preferably sleeve 38 consists of perfluoroalkoxy impregnated with barium sulfate.

A light-scattering component 48 which is filled with a light-scattering material and located on the distal end 39 of the core 31 can reflect light back into the core 31 so as to provide a more even or uniform light distribution. Alexandrite particles can be employed as the light-scattering material for light-scattering component 48. In addition to its light-scattering properties, the light-scattering component 48 fluoresces in a temperature-dependent manner upon being stimulated by light. The fluorescent properties of the alexandrite particles, when stimulated by light energy of the proper wavelength, can determine the temperature of surrounding human tissue employing methods which are known in the art. This temperature-dependent fluorescence property of the light-scattering component 48 is adapted to be used as a temperature sensor 99 in order to measure temperatures in the human tissue in proximity to the light-diffusing section 19.

Preferably, the energy delivery device 12 with connector 28 is the fiberoptic system associated with the Indigo® Optima laser system, which is also commercially available from Ethicon Endo-Surgery Inc. The energy delivery device 12 along with the energy generator 22 are further described and disclosed in U.S. patent No. 6,522,806, entitled "Optical Fiber Including A Diffuser Portion And Continuous Sleeve For The Transmission Of Light" issued to James, IV et al. on February 18, 2003; U.S. patent application Pub. No. 2001/0025173, entitled "Energy Application System With Ancillary Information Exchange Capability, Energy Applicator, And Methods Associated Therewith" by Ritchie et al. and published on September 27, 2001; U.S. patent application Pub. No. 2002/0081871, entitled "Connector Incorporating A Contact Pad Surface On A Plane Parallel To A Longitudinal Axis" by Swayze et al. and published on June 27, 2002; and U.S. patent application Pub. No. 2003/0118302, entitled "Optical Fiber Including A Diffuser Portion And Continuous Sleeve For The Transmission Of Light" by James, IV et al. and published on June 26, 2003, each of which, including the entire disclosures thereof, are hereby incorporated herein by this reference.

Upon connection of the energy delivery device 12 to the energy generator 22, the energy delivery device 12 is ready to receive energy from the energy generator 22 and deliver the energy to the human tissue (not shown) from its light-diffusing section 19 of optical fiber 13.

During operation of the medical instrument 20, light generated by the energy generator 22 travels through the core 31 to the light-diffusing section 19. There, light energy emerges from the core 31 to the optical coupling layer 40 because of the optical coupling layer having a higher index of refraction. The distal portion 44 of the sleeve 38, which surrounds the optical coupling layer 40, collects the light from the optical coupling layer 40. The sleeve 38 preferably uses barium sulfate particles scattered within the sleeve 38 to diffuse light energy evenly outwards towards the human tissue. Light energy reaching the light-scattering component 48 is reflected back towards the core 31 by the alexandrite particles in the light-scattering component 48.

Such light-diffusing section 19 of optical fiber 13 of energy delivery device 12 is used to scatter and diffuse light into human tissue thereby heating the human tissue. It is preferable that the light-diffusing section 19 emit energy into the human tissue in a substantially uniform manner, and as such, the energy is diffused radially outwardly in a uniform distribution along the entire length of the light-diffusing section 19 to assure proper heating of the human tissue being treated.

A common problem is that the light-diffusing section 19 tends to absorb some amount of the emitted light energy. This absorption of light is exhibited by a phenomena known as the self-heating characteristic. This phenomena causes the light-diffusing section 19 to slightly increase in temperature, become hot, or to heat itself up. Although the materials of the optical fibers 13 including the light-diffusing section 19, core 31, optical coupling layer 40, distal portion 44 of sleeve 38 and light-scattering component 48, are selected to minimize absorption of the laser energy, in practical terms, there will always be some small amount of energy self-absorption by these constituent components, either singly or in combination, which results in the self-heating of the light-diffusing section 19. This energy self-absorption property can be caused by the specific design, composition or construction of the various constituents of the energy delivery device 12. Additionally, despite the use of a clean environment and pure materials, contamination can occur during handling or manufacturing of the energy delivery device 12. Contaminants can be trapped within or between the various layers including in the core 31, cladding 32, optical coupling layer 40, light scattering component 48, or sleeve 38. As laser light is diffused or passes through these various constituent layers, some portion of the light energy can be absorbed by the contaminants therein which may cause some self-heating. These self-heating characteristics cause the temperature of that portion of the light-diffusing section 19 of energy delivery device 12 to increase.

Energy self-absorption properties, as used herein, means the degree to which a component, such as the light-diffusing section 19 of optical fiber 13, absorbs some of the energy being delivered through itself, rather than transmitting all of the energy to the human tissue being treated. These energy self-absorption properties can be a function of the materials selected, but also can be a function of power, time, wavelength, or temperature. Since temperature sensor 99 is intended to measure the temperature of the human tissue immediately adjacent to the light-diffusing section 19, this energy self-absorption property represents both an inefficiency in energy transmission that results in a decrease in the therapeutic treatment of the human tissue and an inaccuracy in the actual temperature of the human tissue versus the measured temperature reported by the temperature sensor 99.

Since these energy self-absorption properties are influenced by the design, composition, and/or construction of optical fiber 13, the self-heating characteristic can vary from individual optical fiber 13 to individual optical fiber 13 even when comparing the same type of optical fiber 13 made of the exact same design at the same time and of the same materials. Although the self-heating characteristic of such optical fibers 13 may not be identical, they could correlate to each other in somewhat of a consistent manner or they could vary depending on the power level associated with the energy being passed through the optical fiber 13.

In order to determine the self-heating characteristic the following test was conducted. Optical fibers 13 were placed into a controlled environment in order to obtain a reference or measured temperature. This reference or measured temperature is equivalent to an actual measurement of the internal temperature of the optical fiber 13. The controlled environment used in this example was a temperature-controlled water bath. The water bath was at 40°C. The optical fiber 13 was then inserted into and confined within the controlled environment. Energy was then applied to the energy delivery device 12. Since the self-heating characteristic is also dependent on the power level being applied to the optical fiber 13, the power levels were varied. The specified power level was applied until the measured temperature was stablilized which was defined as the temperature changing less than 0.3°C in a 10 second period. Upon temperature stabilization, the measured temperature was recorded. In this example, the power level was varied from 2 watts to 20 watts (W) of energy. Temperature measurements were taken at five power level settings for each of the five different optical fibers 13 utilized in this test. For example, the measured temperature of the energy delivery device 12 using fiber No. 2 at a power level of 10W in the 40°C water bath is 47°C. In a preferable embodiment of this invention, this measured temperature is desired to be less than 69°C. The resulting temperatures for all five fibers tested are tabulated in Table 1.

Table 1. Power versus Measured Temperature.

| Power | Fiber 1 (°C) | Fiber 2 (°C) | Fiber 3 (°C) | Fiber 4 (°C) | Fiber 5(°C) |
|---|---|---|---|---|---|
| 2W | 39.7 | 40.3 | 40.9 | 38.6 | 40.7 |
| 5W | 44.2 | 43.9 | 46.4 | 43.1 | 47.4 |
| 10W | 48.4 | 47 | 52.5 | 47 | 54.3 |
| 15W | 53.2 | 51.2 | 58.4 | 50.1 | 60.6 |
| 20W | 58.1 | 54.6 | 64.5 | 54.4 | 68.8 |

Self-heating represents the heat due to the amount of energy absorbed by the optical fiber 13. The self-heating characteristic is determined by subtracting the temperature of the controlled environment from the measured temperature reported by the energy delivery device 12. Therefore, in order to determine the actual self-heating characteristic for each of the five optical fibers 13 in this test, the environmental temperature of the 40°C water bath was subtracted from the measured temperature at each of the five power level settings. Consequently, the self-heating characteristic of the optical fiber 13 of the energy delivery device 12 is a function of the specific power level setting of the energy generator 22. The resulting self-heating characteristics for all five fibers tested are tabulated in Table 2. Note that at low power levels; some values appear slightly negative due to standard temperature measurement tolerances (in this case, +/-2°C).

Table 2. Power versus Self-Heating Characteristic.

| Power | Fiber 1(°C) | Fiber 2 (°C) | Fiber 3 (°C) | Fiber 4 (°C) | Fiber 5(°C) |
|---|---|---|---|---|---|
| 2W | -0.3 | 0.3 | 0.9 | -1.4 | 0.7 |
| 5W | 4.2 | 3.9 | 6.4 | 3.1 | 7.4 |
| 10W | 8.4 | 7 | 12.5 | 7 | 14.3 |
| 15W | 13.2 | 11.2 | 18.4 | 10.1 | 20.6 |
| 20W | 18.1 | 14.6 | 24.5 | 14.4 | 28.8 |

After the determination of these self-heating characteristics, calibration parameters indicative of the particular self-heating characteristics for each of the individual optical fibers 13 at each power level can be identified and programmed into memory device 58.

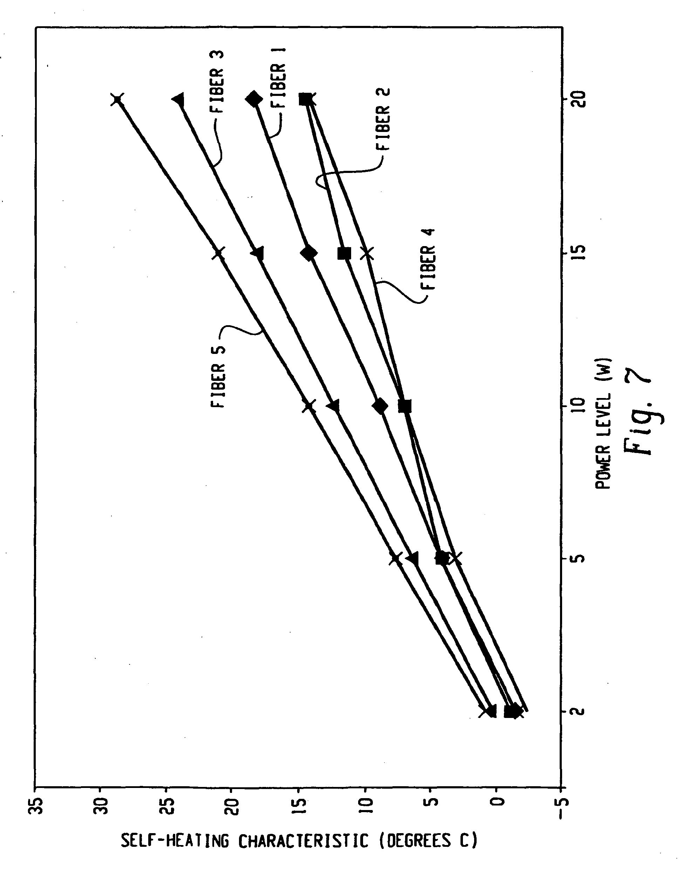

In a similar manner, curves can be fit through the data points corresponding to the self-heating characteristics when plotted against the power levels of Table 2. Such a graphical plot corresponding to Table 2 is shown in FIG. 7. For such a set of curves, functions can be generated that represent each of the particular curves. Such a function could be modeled by a linear equation or even a non-linear equation. In one embodiment, a third-order polynomial could fit the set of curves and the coefficients relating thereto could be identified. For example, in another embodiment, if the self-heating characteristics of the optical fiber 13 are modeled by a linear equation, then the temperature correction performed by energy generator 22 could be:

$$T_a = T_m - (slope)* P + offset$$

Where:

$T_a$ is the actual temperature, °C.
$T_m$ is the measured temperature, °C.
P is the power level at time of measurement, Watts.
Slope and Offset are calibration parameters.

All of these curves, functions, calibration parameters, equations, data points and coefficients are data that is directly related to and indicative of the self-heating characteristic of the optical fiber 13. This data is also specifically associated with the energy self-absorption properties of optical fiber 13 of the energy delivery device 12. Thus, a direct relationship exists between the energy self-absorption properties and the self-heating characteristics. One can result from the other in that the larger the actual temperature value associated with the self-heating characteristic of the optical fiber 13, the greater the energy self-absorption properties. For example, from FIG. 7, one can indicate that fiber No. 5 has greater or more energy self-absorption properties than fiber No. 2 throughout the spectrum of power levels tested because the self-heating characteristic for fiber No. 5 is larger throughout the entire range.

The data and information of various types can be converted into digital information and loaded, stored or programmed into memory device 58 including all of the above identified curves, functions, calibration parameters, equations, data points, coefficients, characteristics, and properties. Self-heating characteristics and energy self-absorption properties of optical fiber 13 along with temperature correction values, and the like can also be programmed into memory device 58. Methods of storing these functions or parameters in digital form are well known in the art.

By way of example, in addition to the usage-related information just described, the data may include information concerning any of the following: identification of the delivering means; expiration, or non-expiration, of the delivering means; parameters for the calibration of the delivering means; the type of energy delivery; operational parameters; energy delivery parameters; monitoring sequence parameters; and any combination thereof. Further by way of example, the data may include information concerning any of the following: identification of the generating means; identification, type, date, or time of treatment; indication or identification of error; amount of energy delivery; integrity of data; and any combination thereof.

Main processor 25 may use the information contained within memory device 58 to automatically modify the energy output of energy generator 22. Also, main processor 25 may make decisions regarding the information contained within memory device 58. For example, main processor 25 may increase or decrease the energy delivered by energy generator 22 based on a particular calibration parameter.

As a further example, main processor 25 may generate error messages and display them on display screen 94 of energy generator 22. For example, an error message may be displayed if the calibration parameter is not detected. Main processor 25 may even write information to memory device 58 to be carried with energy delivery device 12. For example, main processor 25 may write to memory device 58 information concerning the type of treatment, date and time of use of energy delivery device 12, any errors generated, total number of uses for energy delivery device 12, or total energy transmitted through energy delivery device 12.

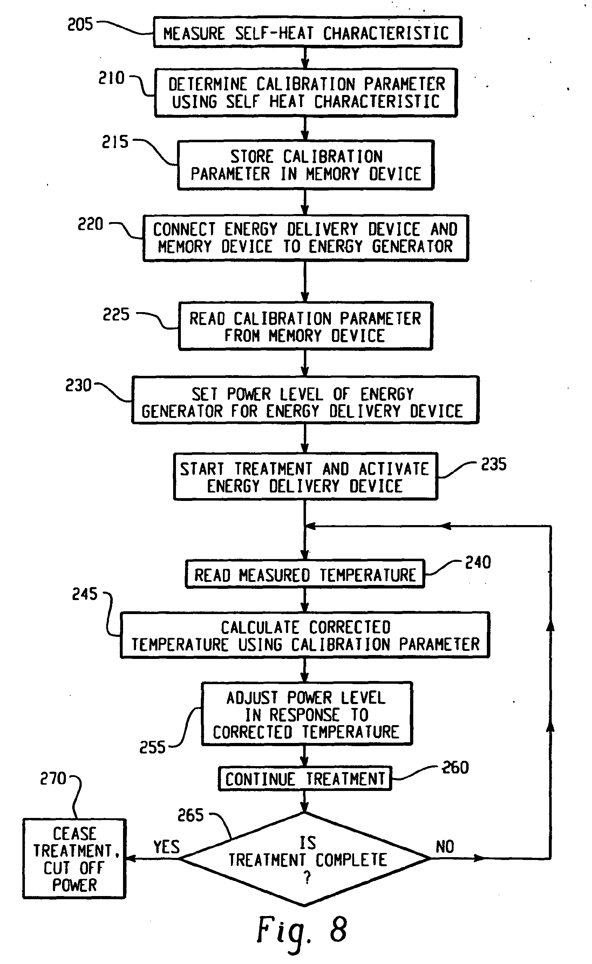

Referring now to FIG. 8, in which a preferred method for using the energy delivery device 12 along with the memory device 58 of the present invention for the treatment of human tissue is shown. During manufacture of the energy delivery device 12, a measurement of the self-heating characteristic 205 of the optical fiber 13 is taken as described previously. Having the self-heating characteristic will enable the determination of a calibration parameter 210. This calibration parameter is for use in memory device 58 and is indicative of the self-heating characteristic which relates directly to the energy self-absorption properties of the particular optical fiber 13. The calibration parameter can be stored as digital information 215 in the memory device by loading or programming the calibration parameter into the memory device 58. Thereafter, energy delivery device 12 has an optical fiber 13 and a memory device 58 that is ready for use in association with energy generator 22 in accordance with the present invention.

The medical treatment system 10 including the energy generator 22 is made ready for use in the treatment of human tissue when it is operatively connected 220 to memory device 58 and energy delivery device 12 as previously described. It will be apparent to those of ordinary skill in the art that the memory device 58 can be a wholly separate unit from the energy delivery device 12 and may even be remotely located and operatively connected to energy generator 22 in a variety of ways known to those skilled in the art. A preferred manner of operatively connecting energy delivery device 12 and memory device 58 to energy generator 22 is by a direct electrical connection. Upon engaging the memory device 58, the energy generator 22 can read the various pieces of information 225 from the memory device 58, including calibration parameters, coefficients, and other data as described previously.

A user of the medical treatment system 10 sets an initial power level 230 on the energy generator 22 for the specific therapeutic use of the energy delivery device 12. Various power levels may be desired based on the type of human tissue to be treated. The user typically starts the treatment 235 by penetrating the human tissue using penetrating tip 50 and positioning light-diffusing section 19 of energy delivery device 12 in a certain location relative to the region of human tissue to be treated. Power is then applied to activate energy delivery device 12.

Temperature sensor 99 measures a temperature at the light-diffusing section 19 of the optical fiber 13. The measured temperature is read 240 for use by main processor 25 of the energy generator 22. The calibration parameter stored within memory device 58 is used by main processor 25 to calculate a corrected temperature value 245 from the measured temperature. Thereafter, the power level of energy generator 22 controlled by the main processor 25 is automatically adjusted 255 in response to the corrected temperature value to assure the proper energy is delivered to the human tissue through energy delivery device 12. The power level is either increased or decreased to the correct power level setting for the desired temperature based on the particular calibration parameter. Treatment continues in this manner 260 assuring efficacious treatment of the human tissue.

Next a determination of whether the treatment is complete or not is to be made 265. Upon determining that the treatment is complete, the treatment is ceased and the power is cut off 270 deactivating energy delivery device 12. If the treatment is determined not to be complete, the treatment continues in a closed-loop manner as illustrated in FIG. 8. This closed-loop control system continues correcting the calculation of the corrected temperature 245 from the measured temperature in order to achieve the most accurate temperature possible. Then this closed-loop system continues to adjust the power level 255 based on that corrected temperature in order to achieve the most precise control of the energy being emitted into the human tissue through the light-diffusing section 19 at the treatment site.

Upon receipt of the continuous signals indicative of the measured temperature of the human tissue from the temperature sensor 99, the main processor 25 of energy generator 22 automatically adjusts the power level to assure that the appropriate amount of energy is diffused through light-diffusing section 19 into the human tissue for the specific medical procedure. This closed-loop control system uses the calibration parameter stored in memory device 58 to monitor and control the power level delivered from the energy generator 22 to the energy delivery device 12 in a continuous real-time process. In this manner the energy delivery device 12 of the present invention is calibrated and reconciles the temperature measurements with the energy delivered to the human tissue by accounting for the self-heating characteristics of the diffusing section 19 of optical fiber 13.

After applying energy to the human tissue and completion of the medical procedure, the user removes connector 28 from connector housing 36. To remove connector 28 the user simply rotates connector 28 from the locked position to the unlocked position. After rotating connector 28, the user pulls on handle portion 88 easily removing connector 28.

The present invention thus provides an efficient medical treatment system 10 having ready capability for the measurement and adjustment or correction of the energy self-absorption properties that result in self-heating characteristics in order to assure accurate, reliable and repeatable human tissue temperature control for more efficacious treatment results during therapeutic procedures. This invention provides an energy delivery device 12 for use in such a system and a method for using the energy delivery device 12.

While preferred embodiments of the present invention have been shown and described herein, it will be understood by those skilled in the art that such embodiments are provided only by way of example. It can be seen by those skilled in the art that embodiments other than those illustrated can make use of the present invention. Numerous variations, modifications, changes, and substitutions may occur to those skilled in the art without departing from this invention. Accordingly, the invention is limited only by the appended claims hereto and the invention is entitled to protection within the full scope of such appended claims.

**Claims**

1. An energy delivery device (12) for use with a medical treatment system (10), wherein the energy delivery device (12) comprises:

An optical fiber (13);

A memory device (58) said memory device (58) having data programmed therein, said data being specifically associated with the energy self-absorption properties of said optical fiber;

Wherein said optical fiber (13) and said memory device (58) are operatively connected to the medical treatment system (10) during use of said medical treatment system;

wherein said data includes a calibration parameter; and **characterized in that**:

said calibration parameter is indicative of a self-heating characteristic of said optical fiber (13).

2. An energy delivery device according to claim 1, wherein said self-heating characteristic is associated with a power level.

3. An energy delivery device according to claim 1, wherein said self-heating characteristic is a function of a power level.

4. An energy delivery device according to claim 3, wherein said function is modeled by an equation.

5. An energy delivery device according to claim 4, wherein said equation is a linear equation.

6. An energy delivery device according to claim 1, further comprising a connector (28) wherein said optical fiber (13) and said memory device (58) are attached to said connector (28).

7. An energy delivery device according to claim 6, wherein said optical fiber (13) has a proximal end and a distal end, said distal end comprising a penetrating tip.

8. An energy delivery device according to claim 7, wherein said optical fiber further comprises a light-diffusing section (19) located adjacent said distal end.

9. An energy delivery device according to claim 8, further comprising a temperature sensor adjacent said light-diffusing section (19).

10. An energy delivery device according to claim 9, wherein said temperature sensor includes alexandrite particles.

11. A memory device (56) for use with an energy delivery device (12) in combination with an optical fiber (13), wherein said memory device (56) comprises:

A memory chip residing on a printed circuit board (66), said memory chip having data programmed therein, said data including a calibration parameter that is specifically associated with the energy self-absorption properties of said optical fiber (13); **characterised in that**:

said calibration parameter is indicative of a self-heating characteristic of said optical fiber (13); and
said memory chip is an electronic erasable programmable read-only memory chip.

**Patentansprüche**

1. Energieabgabegerät (12) zur Verwendung mit einem medizinischen Behandlungssystem (10), wobei das Energieabgabegerät (12) aufweist:

eine optische Faser (13);
eine Speichervorrichtung (58), wobei die Speichervorrichtung (58) programmierte Daten enthält, wobei die Daten speziell mit den Energieselbstabsorptionseigenschaften der optischen Faser verknüpft sind;
wobei die optische Faser (13) und die Speichervorrichtung (58) während der Verwendung des medizinischen Behandlungssystems betrieblich mit dem medizinischen Behandlungssystem (10) verbunden sind;
wobei die Daten einen Kalibrierparameter enthalten; und **dadurch gekennzeichnet, daß**:

der Kalibrierparameter eine Selbstaufheizeigenschaft der optischen Faser (13) anzeigt.

2. Energieabgabegerät nach Anspruch 1, bei dem die Selbstaufheizeigenschaft mit einem Leistungspegel verknüpft ist.

**3.** Energieabgabegerät nach Anspruch 1, bei dem die Selbstaufheizeigenschaft eine Funktion eines Leistungspegels ist.

**4.** Energieabgabegerät nach Anspruch 3, bei dem die Funktion durch eine Gleichung modelliert wird.

**5.** Energieabgabegerät nach Anspruch 4, bei dem die Gleichung eine lineare Gleichung ist.

**6.** Energieabgabegerät nach Anspruch 1, weiter mit einem Verbinder (28), wobei die optische Faser (13) und die Speichervorrichtung (58) an dem Verbinder (28) befestigt sind.

**7.** Energieabgabegerät nach Anspruch 6, bei dem die optische Faser (13) ein proximales und ein distales Ende hat, wobei das distale Ende eine Eindringspitze aufweist.

**8.** Energieabgabegerät nach Anspruch 7, bei dem die optische Faser weiter einen Licht diffundierenden Abschnitt (19) aufweist, der sich benachbart dem distalen Ende befindet.

**9.** Energieabgabegerät nach Anspruch 8, weiter mit einem Temperatursensor benachbart dem Licht diffundierenden Abschnitt (19).

**10.** Energieabgabegerät nach Anspruch 9, bei dem der Temperatursensor Alexandrit-Teilchen umfaßt.

**11.** Speichervorrichtung (58) zur Verwendung mit einem Energieabgabegerät (12) in Kombination mit einer optischen Faser (13), wobei die Speichervorrichtung (58) aufweist:

einen Speicherchip, der sich auf einer gedruckten Schaltkarte (66) befindet, wobei der Speicherchip einprogrammierte Daten enthält, wobei die Daten einen Kalibrierparameter umfassen, der speziell mit den Energieselbstabsorptionseigenschaften der optischen Faser (13) verknüpft ist; **dadurch gekennzeichnet, daß**:

der Kalibrierparameter eine Selbstaufheizeigenschaft der optischen Faser (13) anzeigt; und
der Speicherchip ein elektronisch löschbarer programmierbarer Nur-Lese-Speicherchip ist.

## Revendications

**1.** Dispositif d'alimentation en énergie (12) destiné à un système de traitement médical (10) dans lequel le dispositif d'alimentation en énergie (12) comprend :

une fibre optique (13);
un dispositif de mémoire (58), ledit dispositif de mémoire (58) contenant des données programmées, lesdites données étant spécifiquement associées aux propriétés d'auto-absorption d'énergie de ladite fibre optique, dans lequel ladite fibre optique (13) et ledit dispositif de mémoire (58) fonctionnent connectés au système de traitement médical (10) durant l'utilisation dudit système de traitement médical ;
dans lequel lesdites données comprennent un paramètre de calibrage ; et **caractérisé en ce que** :

ledit paramètre de calibrage est indicatif d'une caractéristique d'auto-chauffage de ladite fibre optique (13).

**2.** Dispositif d'alimentation en énergie selon la revendication 1, dans lequel ladite caractéristique d'auto-chauffage est associée à un niveau de puissance.

**3.** Dispositif d'alimentation en énergie selon la revendication 1, dans lequel ladite caractéristique d'auto-chauffage est fonction d'un niveau de puissance.

**4.** Dispositif d'alimentation en énergie selon la revendication 3, dans lequel ladite fonction est modélisée par une équation.

**5.** Dispositif d'alimentation en énergie selon la revendication 4, dans lequel ladite équation est une équation linéaire.

**6.** Dispositif d'alimentation en énergie selon la revendication 1, comprenant en outre un connecteur (28), ladite fibre optique (13) et ledit dispositif de mémoire (58) étant reliés audit connecteur (28).

7. Dispositif d'alimentation en énergie selon la revendication 6, dans lequel ladite fibre optique (13) a une extrémité proximale et une extrémité distale, ladite extrémité distale comprenant un embout de pénétration.

8. Dispositif d'alimentation en énergie selon la revendication 7, dans lequel ladite fibre optique comprend en outre une section de diffusion de lumière (19) adjacente à ladite extrémité distale.

9. Dispositif d'alimentation en énergie selon la revendication 8, comprenant en outre une sonde de température adjacente à ladite section de diffusion de lumière (19).

10. Dispositif d'alimentation en énergie selon la revendication 9, dans lequel ladite sonde de température comprend des particules d'alexandrite.

11. Dispositif de mémoire (58) destiné à un dispositif d'alimentation en énergie (12) associé à une fibre optique (13) ledit dispositif de mémoire (58) comprenant :

une puce de mémoire résidant sur une carte de circuit imprimé (66), ladite puce de mémoire contenant des données programmées, lesdites données comprenant un paramètre de calibrage qui est spécifiquement associé aux propriétés d'auto-absorption d'énergie de ladite fibre optique (13), **caractérisé en ce que** :

ledit paramètre de calibrage est indicatif d'une caractéristique d'auto-chauffage de ladite fibre optique (13) ; et
ladite puce de mémoire est une puce électronique de mémoire morte effaçable et programmable.

*Fig. 1*

Fig. 2

*Fig. 3*

*Fig. 4*

Fig. 5

Fig. 6

*Fig. 7*

205 — MEASURE SELF-HEAT CHARACTERISTIC

210 — DETERMINE CALIBRATION PARAMETER USING SELF HEAT CHARACTERISTIC

215 — STORE CALIBRATION PARAMETER IN MEMORY DEVICE

220 — CONNECT ENERGY DELIVERY DEVICE AND MEMORY DEVICE TO ENERGY GENERATOR

225 — READ CALIBRATION PARAMETER FROM MEMORY DEVICE

230 — SET POWER LEVEL OF ENERGY GENERATOR FOR ENERGY DELIVERY DEVICE

START TREATMENT AND ACTIVATE ENERGY DELIVERY DEVICE — 235

READ MEASURED TEMPERATURE — 240

245 — CALCULATE CORRECTED TEMPERATURE USING CALIBRATION PARAMETER

ADJUST POWER LEVEL IN RESPONSE TO CORRECTED TEMPERATURE
255 —

CONTINUE TREATMENT — 260

265 — IS TREATMENT COMPLETE ?

YES    NO

270 — CEASE TREATMENT, CUT OFF POWER

*Fig. 8*

**EP 1 535 584 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5074632 A
- US 4695697 A
- US 4476512 A
- US 5057099 A
- US 4564012 A
- US 6522806 B
- US 20010025173 A
- US 20020081871 A
- US 20030118302 A